# EUROPEAN PATENT APPLICATION

(11) **EP 3 950 943 A1**
(43) Date of publication of application: **09.02.2022**
(21) Application number: 20794668.2
(22) Date of filing: 24.04.2020
(51) Int. Cl.: C12N 15/09, C12Q 1/6883, G01N 33/50, G01N 33/53, G01N 33/68

(54) **EYE DISEASE MARKER**

(30) Priority: 26.04.2019 JP 2019085809
(71) Applicant: Senju Pharmaceutical Co., Ltd., Osaka-shi, Osaka 541-0048 (JP)
(72) Inventor: SHIMAZAKI Jun, Tokyo 101-0051 (JP)
(74) Representative: Engelhard, Markus
(86) International application number: PCT/JP2020/017653
(87) International publication number: WO 2020/218488

(57) **Abstract**

The present disclosure provides a marker that shows a relationship with changes in the nerve density and morphology in the cornea. In one aspect, the present invention provides a marker for neuropathy in the eye, the expression of which changes in correlation with morphological parameters of the nerves in the eye. In some embodiments, the parameter may include at least one parameter selected from the group consisting of CNBD, CTBD, CNFD, CNFL, and tortuosity. In a particular embodiment, the parameter may include at least one parameter selected from CNBD and CTBD.

## Description

### [Technical Field]

The present disclosure relates to a marker for a condition of an eye (e.g., nerve abnormality in an eye, dry eye, or the like) and related technologies.

### [Background Art]

Dry eye is defined as "disease that reduces the stability of the tear film due to various factors, which results in ocular discomfort or abnormality in the visual function and is accompanied by a disorder of the ocular surface in some cases" (Non Patent Literature 1 incorporated herein by reference). As defined therein, the causes and symptoms thereof are diverse. The primary therapy was replenishment of moisture with an artificial lachrymal fluid in the past. Meanwhile, with advancements in elucidation of the pathology of dry eye and development of therapeutic drugs, the cause of reduced stability of the tear film is currently diagnosed by layers of the ocular surface (keratoconjunctival epithelium, lachrymal aqueous layer, or oily layer), and a therapy suitable for each case can be proposed (Non Patent Literature 1 incorporated herein by reference). Meanwhile, corneal sensory nerve is an important element for maintaining homeostasis on the ocular surface. A corneal sensory nerve that has branched from the first branch of a trigeminal nerve is distributed throughout the cornea, senses and transmits pain or temperature on the ocular surface to the brain, and promotes blinking or lachrymal fluid secretion to contribute to the maintenance of homeostasis on the ocular surface. Ever since the corneal sensory nerve became observable with a confocal laser scanning microscope, a change in the nerve density or shape in dry eye patients started to be reported (Non Patent Literatures 2 and 3 incorporated herein by reference). Meanwhile, it is still unknown whether such a change is the cause of dry eye.

Although studies that search for a biomarker from lachrymal fluid components from dry eye patients have increased with advancement in proteomics analysis technologies in recent years (Non Patent Literatures 4 and 5), a marker indicating a relationship with a change in the nerve density or shape has not been found. Currently, a therapeutic drug for a corneal sensory nerve is not available.

### [Citation List]

### [Non Patent Literature]

[NPL 1] Japan Dry Eye Society, the Dry Eye Society committee, Revised Definition and Diagnostic Criteria of Dry Eye (2016)
[NPL 2] Cruzat A, Pavan-Langston D, Hamrah P. In vivo confocal microscopy of corneal nerves: analysis and clinical correlation. Semin Ophthalmol. 2010; 25(5-6): 171-7.
[NPL 3] Alhatem A, Cavalcanti B, Hamrah P. In vivo confocal microscopy in dry eye disease and related conditions. Semin Ophthalmol. 2012; 27(5-6): 138-48.
[NPL 4] Srinivasan S, Thangavelu M, Zhang L, et al. iTRAQ quantitative proteomics in the analysis of tears in dry eye patients. Invest Ophthalmol Vis Sci. 2012; 53(8): 5052-9.
[NPL 5] Perumal N, Funke S, Pfeiffer N, et al. Proteomics analysis of human tears from aqueous-deficient and evaporative dry eye patients. Sci Rep. 2016; 6: 29629.

### [Summary of Invention]

### [Solution to Problem]

The inventors newly found a relationship of the morphology of a corneal sensory nerve with corneal sensitivity and a dry eye symptom by observing and measuring the morphology of a corneal sensory nerve of dry eye patients and health adults with a confocal laser scanning microscope, measuring corneal sensitivity, and conducting an ophthalmic study on common dry eye test items, i.e., subjective symptom and ocular surface. As a result of diligent studies, the inventors completed the present invention by finding a marker that indicates a relationship with a change in the nerve density or morphology in the cornea.

Thus, the present disclosure provides the following.

### (Item 1)

A marker for a nerve abnormality in an eye, wherein the marker changes expression in correlation with at least one parameter selected from the group consisting of CNBD, CTBD, CNFD, CNFL, and tortuosity.

### (Item 2)

The marker of item 1, wherein the parameter comprises at least one parameter selected from the group consisting of CNBD and CTBD.

### (Item 3)

The marker of item 1 or 2, wherein the marker is selected from the group consisting of cystatin-S, profilin-1, protein S100-A8, neutrophil gelatinase-associated lipocalin, phospholipid transfer protein, protein S100-A9, lipocalin-1, α1 acidic glycoprotein 1, α1-antichymotrypsin, glyceraldehyde-3-phosphate dehydrogenase, neutrophil elastase, cytokeratin 10 (Keratin, type I cytoskeletal 10), haptoglobin, protein S100-A6, paralemmin-1, laminin α5, SEC14-like protein 1, trypsin-2, secretoglobin family 1D member 1, γ actin (Actin, cytoplasmic 2), α enolase, mammaglobin-B, 14-3-3 protein ζ/δ, epithelial fatty acid binding protein, cytokeratin 75 (Keratin, type II cytoskeletal 75), histone H1.4, keratin 84 (Keratin, type II cuticular Hb4), galectin-7, cathepsin D, cytokeratin 6B (Keratin, type II cytoskeletal 6B), histone H1.1, cytokeratin 17 (Keratin, type I cytoskeletal 17), neutrophil defensin 3, cytokeratin 79 (Keratin, type II cytoskeletal 79), histone H2B type 1-N, peroxiredoxin-1, cytokeratin 12 (Keratin, type I cytoskeletal 12), transcobalamin-1, trypsin-1, lysozyme C, phosphoglycerate kinase 1, cyclophilin A (Peptidyl-prolyl cis-trans isomerase A), and any combination thereof.

### (Item 4)

The marker of any one of items 1 to 3, wherein the marker is selected from the group consisting of cystatin-S, profilin-1, protein S100-A8, neutrophil gelatinase-associated lipocalin, phospholipid transfer protein, protein S100-A9, and any combination thereof.

### (Item 5)

The marker of any one of items 1 to 4, wherein the marker is a marker for a nerve abnormality in dry eye.

### (Item 6)

A detection agent for detecting the marker of any one of items 1 to 5.

### (Item 7)

A method for identifying a marker for a nerve abnormality in an eye, comprising the steps of:
(a) obtaining a parameter selected from the group consisting of CNBD, CTBD, CNFD, CNFL, and tortuosity in a healthy individual and a dry eye patient;
(b) measuring one or more gene expression levels or protein expression levels in a sample obtained from the healthy individual and the dry eye patient; and
(c) correlating the measurement value with the one or more gene expression levels or protein expression levels.

### (Item 8)

The method of item 7, wherein the parameter comprises at least CNBD or CTBD.

### (Item 9)

The method of item 7 or 8, wherein the sample is a lachrymal fluid.

### (Item 10)

The method of any one of items 7 to 9, wherein the marker is a marker for dry eye associated with a nerve abnormality.

### (Item 11)

A marker for dry eye, wherein the marker changes expression in correlation with at least one parameter selected from the group consisting of BUT, OSDI, and DEQ5.

### (Item 12)

The marker of item 11, selected from the group consisting of 14-3-3 protein θ, secretory leukocyte protease inhibitor (Antileukoproteinase), galectin-3-binding protein, α actin 1 (Actin, alpha skeletal muscle), α1-antichymotrypsin, calmodulin-3, cathepsin D, epithelial fatty acid binding protein, fibrinogen β chain, glucose-6-phosphate isomerase, histone H1.4, histone H2A type 1-C, histone H2B type 1-J, keratin 84 (Keratin, type II cuticular Hb4), cytokeratin 79 (Keratin, type II cytoskeletal 79), laminin α5, non-histone chromosomal protein HMG-17, nucleobindin-2, peroxiredoxin-1, polymeric immunoglobulin receptor, protein S100-A7, SEC14-like protein 1, serotransferrin, thioredoxin, trypsin-2, trypsin-3, and any combination thereof.

### (Item 13)

The marker of item 11 or 12, wherein the marker is selected from the group consisting of 14-3-3 protein θ, secretory leukocyte protease inhibitor (Antileukoproteinase), galectin-3-binding protein, and any combination thereof.

### (Item 14)

A detection agent for detecting the marker of any one of items 11 to 13.

### (Item 15)

Markers for dry eye associated with a nerve abnormality, comprising at least one marker of any one of items 1 to 5 and at least one marker of any one of items 11 to 13.

### (Item 1A)

A method of diagnosing whether a subject has a nerve abnormality in an eye, the method comprising the steps of:
obtaining a sample of the subject;
measuring an amount of a marker for a nerve abnormality in an eye in the sample, wherein the marker is a marker, wherein the marker changes expression in correlation with at least one parameter selected from the group consisting of CNBD, CTBD, CNFD, CNFL, and tortuosity; and
comparing the measured amount of the marker with an amount of the marker in a sample obtained from a healthy individual to determine that the subject has a nerve abnormality in an eye based on the measured amount of the marker.

### (Item 2A)

The method of item 1A, wherein the measured amount of the marker that is higher or lower than the amount of the marker in the sample obtained from the healthy individual can result in a diagnosis of having, or likely having, a nerve abnormality.

### (Item 3A)

The method of item 1A or 2A, wherein the parameter comprises at least one parameter selected from the group consisting of CNBD and CTBD.

### (Item 4A)

The method of any one of items 1A to 3A, wherein the marker is selected from the group consisting of cystatin-S, profilin-1, protein S100-A8, neutrophil gelatinase-associated lipocalin, phospholipid transfer protein, protein S100-A9, lipocalin-1, α1 acidic glycoprotein 1, α1-antichymotrypsin, glyceraldehyde-3-phosphate dehydrogenase, neutrophil elastase, cytokeratin 10 (Keratin, type I cytoskeletal 10), haptoglobin, protein S100-A6, paralemmin-1, laminin α5, SEC14-like protein 1, trypsin-2, secretoglobin family 1D member 1, γ actin (Actin, cytoplasmic 2), α enolase, mammaglobin-B, 14-3-3 protein ζ/δ, epithelial fatty acid binding protein, cytokeratin 75 (Keratin, type II cytoskeletal 75), histone H1.4, keratin 84 (Keratin, type II cuticular Hb4), galectin-7, cathepsin D, cytokeratin 6B (Keratin, type II cytoskeletal 6B), histone H1.1, cytokeratin 17 (Keratin, type I cytoskeletal 17), neutrophil defensin 3, cytokeratin 79 (Keratin, type II cytoskeletal 79), histone H2B type 1-N, peroxiredoxin-1, cytokeratin 12 (Keratin, type I cytoskeletal 12), transcobalamin-1, trypsin-1, lysozyme C, phosphoglycerate kinase 1, cyclophilin A (Peptidyl-prolyl cis-trans isomerase A), and any combination thereof.

### (Item 5A)

The method of any one of items 1A to 4A, wherein the marker is selected from the group consisting of cystatin-S, profilin-1, protein S100-A8, neutrophil gelatinase-associated lipocalin, phospholipid transfer protein, protein S100-A9, and any combination thereof.

### (Item 6A)

The method of any one of items 1A to 5A, wherein the marker is a marker for a nerve abnormality in dry eye.

### (Item 7A)

The method of any one of items 1A to 6A, wherein the sample is a lachrymal fluid.

### (Item 8A)

A method of diagnosing whether a subject has dry eye, the method comprising the steps of:
obtaining a sample of the subject;
measuring an amount of a marker for dry eye in the sample, wherein the marker is a marker, wherein the marker changes expression in correlation with at least one parameter selected from the group consisting of BUT, OSDI, and DEQ5; and
comparing the measured amount of the marker with an amount of the marker in a sample obtained from a healthy individual to determine that the subject has dry eye based on the measured amount of the marker.

### (Item 9A)

The method of item 8A, wherein the measured amount of the marker that is higher or lower than the amount of the marker in the sample obtained from the healthy individual can result in a diagnosis of having, or likely having, a nerve abnormality.

### (Item 10A)

The method of item 8A or 9A, wherein the marker is selected from the group consisting of 14-3-3 protein θ, secretory leukocyte protease inhibitor (Antileukoproteinase), galectin-3-binding protein, α actin 1 (Actin, alpha skeletal muscle), α1-antichymotrypsin, calmodulin-3, cathepsin D, epithelial fatty acid binding protein, fibrinogen β chain, glucose-6-phosphate isomerase, histone H1.4, histone H2A type 1-C, histone H2B type 1-J, keratin 84 (Keratin, type II cuticular Hb4), cytokeratin 79 (Keratin, type II cytoskeletal 79), laminin α5, non-histone chromosomal protein HMG-17, nucleobindin-2, peroxiredoxin-1, polymeric immunoglobulin receptor, protein S100-A7, SEC14-like protein 1, serotransferrin, thioredoxin, trypsin-2, trypsin-3, and any combination thereof.

### (Item 11A)

The method of any one of items 8A to 10A, wherein the marker is selected from the group consisting of 14-3-3 protein θ, secretory leukocyte protease inhibitor (Antileukoproteinase), galectin-3-binding protein, and any combination thereof.

### (Item 12A)

The method of any one of items 8A to 11A, wherein the sample is a lachrymal fluid.

### (Item 13A)

A method of diagnosing whether a subject has dry eye associated with a nerve abnormality, the method comprising the steps of:
obtaining a sample from the subject;
measuring at least one marker specified in any one of items 1A to 7A and at least one marker specified in any one of items 8A to 12A in the sample; and
comparing the measured amount of the marker with an amount of the marker in a sample obtained from a healthy individual to determine that the subject has dry eye associated with a nerve abnormality based on the amount of the marker.

### (Item 1B)

A method of treating an eye in a subject, the method comprising the steps of:
obtaining a sample of the subject;
measuring an amount of a marker for a nerve abnormality in an eye in the sample, wherein the marker is a marker, wherein the marker changes expression in correlation with at least one parameter selected from the group consisting of CNBD, CTBD, CNFD, CNFL, and tortuosity;
comparing the measured amount of the marker with an amount of the marker in a sample obtained from a healthy individual to diagnose whether the subject has a nerve abnormality in the eye; and
administering a drug for preventing or treating nerve abnormality to a subject determined as having the nerve abnormality in the step of diagnosing.

### (Item 2B)

The method of item 1B, wherein the measured amount of the marker that is higher or lower than the amount of the marker in the sample obtained from the healthy individual can result in a diagnosis of having, or likely having, a nerve abnormality.

### (Item 3B)

The method of item 1B or 2B, wherein the parameter comprises at least one parameter selected from the group consisting of CNBD and CTBD.

### (Item 4B)

The method of any one of items 1B to 3B, wherein the marker is selected from the group consisting of cystatin-S, profilin-1, protein S100-A8, neutrophil gelatinase-associated lipocalin, phospholipid transfer protein, protein S100-A9, lipocalin-1, α1 acidic glycoprotein 1, α1-antichymotrypsin, glyceraldehyde-3-phosphate dehydrogenase, neutrophil elastase, cytokeratin 10 (Keratin, type I cytoskeletal 10), haptoglobin, protein S100-A6, paralemmin-1, laminin α5, SEC14-like protein 1, trypsin-2, secretoglobin family 1D member 1, γ actin (Actin, cytoplasmic 2), α enolase, mammaglobin-B, 14-3-3 protein ζ/δ, epithelial fatty acid binding protein, cytokeratin 75 (Keratin, type II cytoskeletal 75), histone H1.4, keratin 84 (Keratin, type II cuticular Hb4), galectin-7, cathepsin D, cytokeratin 6B (Keratin, type II cytoskeletal 6B), histone H1.1, cytokeratin 17 (Keratin, type I cytoskeletal 17), neutrophil defensin 3, cytokeratin 79 (Keratin, type II cytoskeletal 79), histone H2B type 1-N, peroxiredoxin-1, cytokeratin 12 (Keratin, type I cytoskeletal 12), transcobalamin-1, trypsin-1, lysozyme C, phosphoglycerate kinase 1, cyclophilin A (Peptidyl-prolyl cis-trans isomerase A), and any combination thereof.

### (Item 5B)

The method of any one of items 1B to 4B, wherein the marker is selected from the group consisting of cystatin-S, profilin-1, protein S100-A8, neutrophil gelatinase-associated lipocalin, phospholipid transfer protein, protein S100-A9, and any combination thereof.

### (Item 6B)

The method of any one of items 1B to 5B, wherein the marker is a marker for a nerve abnormality in dry eye.

### (Item 7B)

The method of any one of items 1B to 6B, wherein the sample is a lachrymal fluid.

### (Item 8B)

A method of treating dry eye in a subject, the method comprising the steps of:
obtaining a sample of the subject;
measuring an amount of a marker for dry eye in the sample, wherein the marker is a marker, wherein the marker changes expression in correlation with at least one parameter selected from the group consisting of BUT, OSDI, and DEQ5;
comparing the measured amount of the marker with an amount of the marker in a sample obtained from a healthy individual to determine that the subject has dry eye based on the amount of the marker; and
administering a drug for preventing or treating dry eye to a subject determined as having dry eye in the step of diagnosing.

### (Item 9B)

The method of item 8B, wherein the measured amount of the marker that is higher or lower than the amount of the marker in the sample obtained from the healthy individual can result in a diagnosis of having, or likely having, a nerve abnormality.

### (Item 10B)

The method of item 8B or 9B, wherein the marker is selected from the group consisting of 14-3-3 protein θ, secretory leukocyte protease inhibitor (Antileukoproteinase), galectin-3-binding protein, α actin 1 (Actin, alpha skeletal muscle), α1-antichymotrypsin, calmodulin-3, cathepsin D, epithelial fatty acid binding protein, fibrinogen β chain, glucose-6-phosphate isomerase, histone H1.4, histone H2A type 1-C, histone H2B type 1-J, keratin 84 (Keratin, type II cuticular Hb4), cytokeratin 79 (Keratin, type II cytoskeletal 79), laminin α5, non-histone chromosomal protein HMG-17, nucleobindin-2, peroxiredoxin-1, polymeric immunoglobulin receptor, protein S100-A7, SEC14-like protein 1, serotransferrin, thioredoxin, trypsin-2, trypsin-3, and any combination thereof.

### (Item 11B)

The method of any one of items 8B to 10B, wherein the marker is selected from the group consisting of 14-3-3 protein θ, secretory leukocyte protease inhibitor (Antileukoproteinase), galectin-3-binding protein, and any combination thereof.

### (Item 12B)

The method of any one of items 8B to 11B, wherein the sample is a lachrymal fluid.

### (Item 13B)

A method of treating dry eye associated with a nerve abnormality by a subject, the method comprising the steps of:
obtaining a sample from the subject;
measuring at least one marker specified in any one of items 1B to 7B and at least one marker specified in any one of items 8B to 12B in the sample;
comparing the measured amount of the marker with an amount of the marker in a sample obtained from a healthy individual to determine that the subject has dry eye associated with a nerve abnormality based on the amount of the marker; and
administering a drug for preventing or treating dry eye associated with a nerve abnormality to a subject determined as having dry eye associated with a nerve abnormality in the step of diagnosing.

### (Item 1C)

An in vitro method of diagnosing whether a subject has a nerve abnormality in an eye, the method comprising the steps of:
measuring a marker for a nerve abnormality in an eye in the sample of the subject in vitro, wherein the marker is a marker, wherein the marker changes expression in correlation with at least one parameter selected from the group consisting of CNBD, CTBD, CNFD, CNFL, and tortuosity; and
comparing the measured amount of the marker with an amount of the marker in a sample obtained from a healthy individual to determine that the subject has a nerve abnormality in an eye based on the amount of the marker.

### (Item 2C)

The method of item 1C, wherein the measured amount of the marker that is higher or lower than the amount of the marker in the sample obtained from the healthy individual can result in a diagnosis of having, or likely having, a nerve abnormality.

### (Item 3C)

The method of item 1C or 2C, wherein the parameter comprises at least one parameter selected from the group consisting of CNBD and CTBD.

### (Item 4C)

The method of any one of items 1C to 3C, wherein the marker is selected from the group consisting of cystatin-S, profilin-1, protein S100-A8, neutrophil gelatinase-associated lipocalin, phospholipid transfer protein, protein S100-A9, lipocalin-1, α1 acidic glycoprotein 1, α1-antichymotrypsin, glyceraldehyde-3-phosphate dehydrogenase, neutrophil elastase, cytokeratin 10 (Keratin, type I cytoskeletal 10), haptoglobin, protein S100-A6, paralemmin-1, laminin α5, SEC14-like protein 1, trypsin-2, secretoglobin family 1D member 1, γ actin (Actin, cytoplasmic 2), α enolase, mammaglobin-B, 14-3-3 protein ζ/δ, epithelial fatty acid binding protein, cytokeratin 75 (Keratin, type II cytoskeletal 75), histone H1.4, keratin 84 (Keratin, type II cuticular Hb4), galectin-7, cathepsin D, cytokeratin 6B (Keratin, type II cytoskeletal 6B), histone H1.1, cytokeratin 17 (Keratin, type I cytoskeletal 17), neutrophil defensin 3, cytokeratin 79 (Keratin, type II cytoskeletal 79), histone H2B type 1-N, peroxiredoxin-1, cytokeratin 12 (Keratin, type I cytoskeletal 12), transcobalamin-1, trypsin-1, lysozyme C, phosphoglycerate kinase 1, cyclophilin A (Peptidyl-prolyl cis-trans isomerase A), and any combination thereof.

### (Item 5C)

The method of any one of items 1C to 4C, wherein the marker is selected from the group consisting of cystatin-S, profilin-1, protein S100-A8, neutrophil gelatinase-associated lipocalin, phospholipid transfer protein, protein S100-A9, and any combination thereof.

### (Item 6C)

The method of any one of items 1C to 5C, wherein the marker is a marker for a nerve abnormality in dry eye.

### (Item 7C)

The method of any one of items 1C to 6C, wherein the sample is a lachrymal fluid.

### (Item 8C)

An in vitro method of diagnosing whether a subject has dry eye, the method comprising the steps of:
measuring a marker for dry eye in a sample of the subject in vitro, wherein the marker is a marker, wherein the marker changes expression in correlation with at least one parameter selected from the group consisting of BUT, OSDI, and DEQ5; and
comparing the measured amount of the marker with an amount of the marker in a sample obtained from a healthy individual to determine that the subject has dry eye in an eye based on the amount of the marker.

### (Item 9C)

The method of item 8C, wherein the measured amount of the marker that is higher or lower than the amount of the marker in the sample obtained from the healthy individual can result in a diagnosis of having, or likely having, a nerve abnormality.

### (Item 10C)

The method of item 8C or 9C, wherein the marker is selected from the group consisting of 14-3-3 protein θ, secretory leukocyte protease inhibitor (Antileukoproteinase), galectin-3-binding protein, α actin 1 (Actin, alpha skeletal muscle), α1-antichymotrypsin, calmodulin-3, cathepsin D, epithelial fatty acid binding protein, fibrinogen β chain, glucose-6-phosphate isomerase, histone H1.4, histone H2A type 1-C, histone H2B type 1-J, keratin 84 (Keratin, type II cuticular Hb4), cytokeratin 79 (Keratin, type II cytoskeletal 79), laminin α5, non-histone chromosomal protein HMG-17, nucleobindin-2, peroxiredoxin-1, polymeric immunoglobulin receptor, protein S100-A7, SEC14-like protein 1, serotransferrin, thioredoxin, trypsin-2, trypsin-3, and any combination thereof.

### (Item 11C)

The method of any one of items 8C to 10C, wherein the marker is selected from the group consisting of 14-3-3 protein θ, secretory leukocyte protease inhibitor (Antileukoproteinase), galectin-3-binding protein, and any combination thereof.

### (Item 12C)

The method of any one of items 8C to 11C, wherein the sample is a lachrymal fluid.

### (Item 13C)

A method of diagnosing whether a subject has dry eye associated with a nerve abnormality, the method comprising the steps of:
obtaining a sample from the subject;
measuring at least one marker specified in any one of items 1C to 7C and at least one marker specified in any one of items 8C to 12C in the sample; and
comparing the measured amount of the marker with an amount of the marker in a sample obtained from a healthy individual to determine that the subject has dry eye based on the measured amount of the marker.

### (Item 1D)

A method of detecting a nerve abnormality in an eye of a subject, the method comprising the steps of:
measuring an amount of a marker for a nerve abnormality in an eye in a sample obtained from the subject, wherein the marker is a marker, wherein the marker changes expression in correlation with at least one parameter selected from the group consisting of CNBD, CTBD, CNFD, CNFL, and tortuosity; and
comparing the measured amount of the marker with an amount of the marker in a sample obtained from a healthy individual.

### (Item 2D)

The method of item 1D, wherein the parameter comprises at least one parameter selected from the group consisting of CNBD and CTBD.

### (Item 3D)

The method of item 1D or 2D, wherein the marker is selected from the group consisting of cystatin-S, profilin-1, protein S100-A8, neutrophil gelatinase-associated lipocalin, phospholipid transfer protein, protein S100-A9, lipocalin-1, α1 acidic glycoprotein 1, α1-antichymotrypsin, glyceraldehyde-3-phosphate dehydrogenase, neutrophil elastase, cytokeratin 10 (Keratin, type I cytoskeletal 10), haptoglobin, protein S100-A6, paralemmin-1, laminin α5, SEC14-like protein 1, trypsin-2, secretoglobin family 1D member 1, γ actin (Actin, cytoplasmic 2), α enolase, mammaglobin-B, 14-3-3 protein ζ/δ, epithelial fatty acid binding protein, cytokeratin 75 (Keratin, type II cytoskeletal 75), histone H1.4, keratin 84 (Keratin, type II cuticular Hb4), galectin-7, cathepsin D, cytokeratin 6B (Keratin, type II cytoskeletal 6B), histone H1.1, cytokeratin 17 (Keratin, type I cytoskeletal 17), neutrophil defensin 3, cytokeratin 79 (Keratin, type II cytoskeletal 79), histone H2B type 1-N, peroxiredoxin-1, cytokeratin 12 (Keratin, type I cytoskeletal 12), transcobalamin-1, trypsin-1, lysozyme C, phosphoglycerate kinase 1, cyclophilin A (Peptidyl-prolyl cis-trans isomerase A), and any combination thereof.

### (Item 4D)

The method of any one of items 1D to 3D, wherein the marker is selected from the group consisting of cystatin-S, profilin-1, protein S100-A8, neutrophil gelatinase-associated lipocalin, phospholipid transfer protein, protein S100-A9, and any combination thereof.

### (Item 5D)

The method of any one of items 1D to 4D, wherein the marker is a marker for a nerve abnormality in dry eye.

### (Item 6D)

The method of any one of items 1D to 5D, wherein the sample is a lachrymal fluid.

### (Item 7D)

A method for detecting dry eye in a subject, the method comprising the steps of:
measuring an amount of a marker for dry eye in the sample, wherein the marker is a marker, wherein the marker changes expression in correlation with at least one parameter selected from the group consisting of BUT, OSDI, and DEQ5; and
comparing the measured amount of the marker with an amount of the marker in a sample obtained from a healthy individual.

### (Item 8D)

The method of item 7D, wherein the marker is selected from the group consisting of 14-3-3 protein θ, secretory leukocyte protease inhibitor (Antileukoproteinase), galectin-3-binding protein, α actin 1 (Actin, alpha skeletal muscle), α1-antichymotrypsin, calmodulin-3, cathepsin D, epithelial fatty acid binding protein, fibrinogen β chain, glucose-6-phosphate isomerase, histone H1.4, histone H2A type 1-C, histone H2B type 1-J, keratin 84 (Keratin, type II cuticular Hb4), cytokeratin 79 (Keratin, type II cytoskeletal 79), laminin α5, non-histone chromosomal protein HMG-17, nucleobindin-2, peroxiredoxin-1, polymeric immunoglobulin receptor, protein S100-A7, SEC14-like protein 1, serotransferrin, thioredoxin, trypsin-2, trypsin-3, and any combination thereof.

### (Item 9D)

The method of item 7D or 8D, wherein the marker is selected from the group consisting of 14-3-3 protein θ, secretory leukocyte protease inhibitor (Antileukoproteinase), galectin-3-binding protein, and any combination thereof.

### (Item 10D)

The method of any one of items 7D to 9D, wherein the sample is a lachrymal fluid.

### (Item 11D)

A method of detecting dry eye associated with a nerve abnormality in a subject, the method comprising the steps of:
measuring at least one marker specified in any one of items 1D to 6D and at least one marker specified in any one of items 7D to 10D in the sample; and
comparing the measured amount of the marker with an amount of the marker in a sample obtained from a healthy individual.

The present invention is intended so that one or more of the features described above can be provided not only as the explicitly disclosed combinations, but also as other combinations thereof. Additional embodiments and advantages of the present invention are recognized by reading and understanding the following detailed description as needed.

### [Advantageous Effects of Invention]

The marker of the present disclosure can be an indicator of a nerve abnormality in an eye. The present disclosure can diagnose a condition associated with a nerve abnormality in an eye and screen for an agent that is effect for therapy of a nerve abnormality in an eye.

### [Brief Description of Drawings]

[Figure 1] Figure **1** shows the schematics of the design of the study in Example 1.
[Figure 2] Figure **2** shows the background of a subject subjected to the study in Example 1.
[Figure 3] Figure **3** shows results of a questionnaire related to subjective symptoms.
[Figure 4] Figure **4** shows results of visual analog scale (VAS) related to dry eye symptoms (blurred vision, photophobia, and eye irritancy).
[Figure 5] Figure **5** shows results of an ophthalmic test.
[Figure 6] Figure **6** shows results of each nerve parameter measured by confocal laser scanning microscopy. Statistical processing used Welch's t-test.
[Figure 7] Figure **7** shows correlation graphs between CNBD or CTBD and BUT. Statistical processing was performed with respect to a Pearson correlation coefficient.
[Figure 8] Figure **8** shows a list of proteins detected in a lachrymal fluid, which had a correlation with a nerve parameter.
[Figure 9] Figure **9** shows correlation graphs between cystatin-S and CNFD, CNBD, CNFL, CTBD, and tortuosity. Statistical processing was performed with respect to a Pearson correlation coefficient.
[Figure 10] Figure **10** shows correlation between profilin-1, protein S100-A8, neutrophil gelatinase-associated lipocalin, phospholipid transfer protein, and protein S100-A9 and each parameter. Statistical processing was performed with respect to a Pearson correlation coefficient.
[Figure 11] Figure **11** shows a list of proteins correlated with BUT or a subjective symptom (DEQ5 and OSDI).
[Figure 12] Figure **12** shows correlation between secretory leukocyte protease inhibitor (Antileukoproteinase), and galectin-3-binding protein and each of the parameters BUT, OSDI and DEQ5. Statistical processing was performed with respect to a Pearson correlation coefficient.

### [Description of Embodiments]

The present invention is described hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence. As used herein, "about" refers to a range of ± 10% of the subsequent value.

### (Definitions)

As used herein, "nerve abnormality" in an eye refers to an abnormality of a corneal sensory nerve. A corneal sensory nerve is a trigeminal nerve. A nerve abnormality in an eye is accompanied with a symptom due to diminished function of a trigeminal nerve. Examples of such a symptom include photophobia, sensation of hotness, reduced sensation of coldness, burning sensation in the eye, ocular pain due to wind, light, and change in temperature, reduced pain tolerance, and the like.

As used herein, "corneal nerve fibre density" (CNFD) refers to the number of major nerve fibers per 1 mm² in the cornea.

As used herein, "corneal nerve branch density" (CNBD) refers to the number of branches on the major nerve fiber per 1 mm² in the cornea.

As used herein, "corneal nerve fibre length" (CNFL) refers to the total length of nerve fiber per 1 mm² in the cornea.

As used herein, "corneal nerve fibre total branch density" (CTBD) refers to the total number of branches per 1 mm² in the cornea.

As used herein, "corneal nerve fibre area" (CNFA) refers to the total area (mm²) of nerve fibers per 1 mm² in the cornea.

As used herein, "corneal nerve fibre width" (CNFW) refers to the mean nerve fiber width (mm) per 1 mm² in the cornea.

As used herein, "nerve tortuosity" refers to the mean curvature of the major fiber of the nerve in the cornea. Tortuosity is a measurement based on SLD or TCI. SLD is a value found from dividing the linear length from the starting point to the end point of the major fiber by the actual length of the major fiber minus 1. TCI refers to the change in the tilt of the main fiber.

As used herein, "marker" refers to an indicator that can be detected in a sample, such as a predictive, diagnostic, and/or prognostic indicator, including polynucleotides (e.g., DNA and/or RNA), proteins, and polypeptides. A marker can serve the role of an indicator of a specific subtype of a disease or disorder characterized by a specific molecular, pathological, histological, and/or clinical property.

As used herein, "dry eye" refers to a condition associated with ocular discomfort or visual abnormality resulting from reduced lachrymal fluid, or change in the component of lachrymal fluid.

As used herein, "subject" refers to a target of administration of the therapeutic or prophylactic composition, combination, or method of the present disclosure. Examples of subjects include mammals (e.g., humans, mice, rats, hamsters, rabbits, cats, dogs, cows, horses, sheep, monkeys, goats, pigs, and the like).

The preferred embodiments are described hereinafter. It is understood that the embodiments are exemplification of the present invention, so that the scope of the present invention is not limited to such preferred embodiments. It should be understood that those skilled in the art can refer to the following preferred embodiments to readily make modifications or changes within the scope of the present invention. Any of these embodiments can be appropriately combined by those skilled in the art.

### (Nerve abnormality marker)

In one embodiment, the present disclosure provides a marker for a nerve abnormality in an eye, which changes (increases or decreases) expression in correlation with a morphological parameter of a nerve in an eye. The inventors found a correlation between a dry eye patient and a morphological parameter of a nerve in an eye (e.g., CNBD, CTBD, CNFD, CNFL, and tortuosity). In particular, CNBD and CTBD were significantly higher in dry eye patients compared to healthy individuals. Thus, in some embodiments, the parameter can comprise at least one parameter selected from the group consisting of CNBD, CTBD, CNFD, CNFL, and tortuosity. In a particular embodiment, the parameter can comprise at least one parameter selected from the group consisting of CNBD and CTBD.

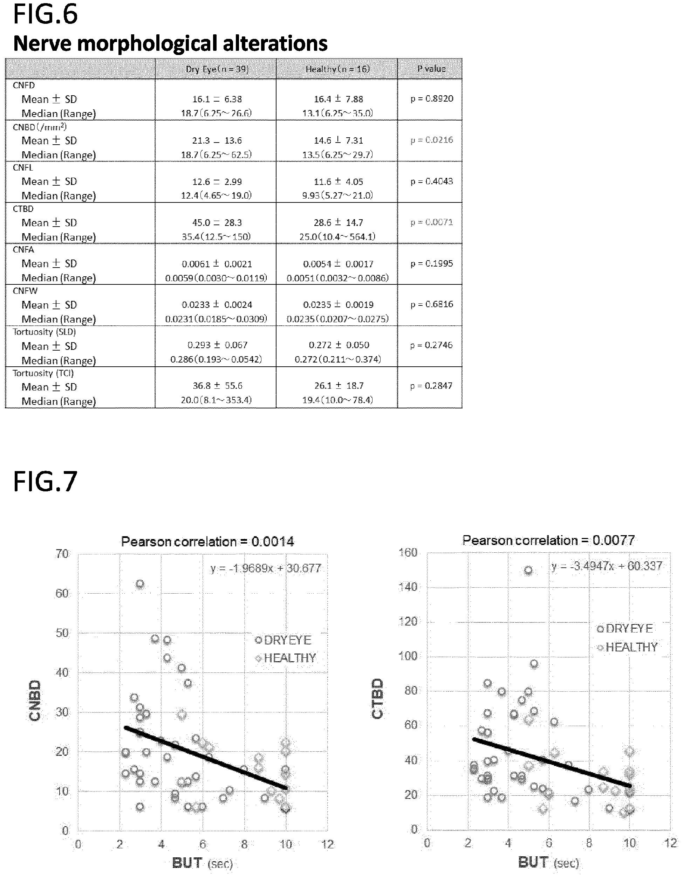

In some embodiments, a nerve abnormality can be a nerve abnormality in the cornea. In a specific embodiment, a nerve abnormality can be a nerve abnormality in dry eye. As shown in Figure **7****,** there are healthy individuals with BUT of 5 seconds and high CNBD and CTBD. Such subjects are not a dry eye patient, but can be a target of therapy as an individual with a nerve abnormality.

In some embodiments, the marker can be selected from the group consisting of cystatin-S, profilin-1, protein S100-A8, neutrophil gelatinase-associated lipocalin, phospholipid transfer protein, protein S100-A9, lipocalin-1, α1 acidic glycoprotein 1, α1-antichymotrypsin, glyceraldehyde-3-phosphate dehydrogenase, neutrophil elastase, cytokeratin 10 (Keratin, type I cytoskeletal 10), haptoglobin, protein S100-A6, paralemmin-1, laminin α5, SEC14-like protein 1, trypsin-2, secretoglobin family 1D member 1, γ actin (Actin, cytoplasmic 2), α enolase, mammaglobin-B, 14-3-3 protein ζ/δ, epithelial fatty acid binding protein, cytokeratin 75 (Keratin, type II cytoskeletal 75), histone H1.4, keratin 84 (Keratin, type II cuticular Hb4), galectin-7, cathepsin D, cytokeratin 6B (Keratin, type II cytoskeletal 6B), histone H1.1, cytokeratin 17 (Keratin, type I cytoskeletal 17), neutrophil defensin 3, cytokeratin 79 (Keratin, type II cytoskeletal 79), histone H2B type 1-N, peroxiredoxin-1, cytokeratin 12 (Keratin, type I cytoskeletal 12), transcobalamin-1, trypsin-1, lysozyme C, phosphoglycerate kinase 1, cyclophilin A (Peptidyl-prolyl cis-trans isomerase A), and any combination thereof.

In a preferred embodiment, the marker can be selected from the group consisting of cystatin-S, profilin-1, protein S100-A8, neutrophil gelatinase-associated lipocalin, phospholipid transfer protein, protein S100-A9, and any combination thereof. These markers are correlated with both CNBD and CTBD.

### (Method of identifying a marker)

In another embodiment, the present disclosure provides a method for identifying a marker for a nerve abnormality in an eye, comprising the steps of: (a) obtaining a parameter selected from the group consisting of CNBD, CTBD, CNFD, CNFL, and tortuosity in a healthy individual and a dry eye patient; (b) measuring one or more gene expression levels or protein expression levels in a sample obtained from the healthy individual and the dry eye patient; and (c) correlating the measurement value with the one or more gene expression levels or protein expression levels.

In some embodiment, the parameter can comprise at least one of CNBD and CTBD. In some embodiment, the sample is a lachrymal fluid. In some embodiments, a nerve abnormality in an eye can be a nerve abnormality in the cornea and/or nerve abnormality in dry eye.

### (Dry eye marker)

In another embodiment, the present disclosure provides a marker for dry eye, which changes (increases or decreases) expression in correlation with at least one parameter selected from the group consisting of BUT, OSDI, and DEQ5.

In some embodiments, the marker can be selected from the group consisting of 14-3-3 protein θ, secretory leukocyte protease inhibitor (Antileukoproteinase), galectin-3-binding protein, α actin 1 (Actin, alpha skeletal muscle), α1-antichymotrypsin, calmodulin-3, cathepsin D, epithelial fatty acid binding protein, fibrinogen β chain, glucose-6-phosphate isomerase, histone H1.4, histone H2A type 1-C, histone H2B type 1-J, keratin 84 (Keratin, type II cuticular Hb4), cytokeratin 79 (Keratin, type II cytoskeletal 79), laminin α5, non-histone chromosomal protein HMG-17, nucleobindin-2, peroxiredoxin-1, polymeric immunoglobulin receptor, protein S100-A7, SEC14-like protein 1, serotransferrin, thioredoxin, trypsin-2, trypsin-3, and any combination thereof.

In a preferred embodiment, the marker can be selected from the group consisting of 14-3-3 protein θ, secretory leukocyte protease inhibitor (Antileukoproteinase), galectin-3-binding protein, and any combination thereof.

In some embodiments, at least one of the markers for a nerve abnormality in an eye described above and at least one of the markers for dry eye described above can be used in combination.

### (Detection agent)

In another embodiment, the present disclosure provides a detection agent for detecting a protein or nucleic acid, which changes (increases or decreases) expression in correlation with a morphological parameter of a nerve in an eye. In some embodiment, a morphological parameter of a nerve is selected from the group consisting of CNBD, CTBD, CNFD, CNFL, and tortuosity, and preferably comprises at least one parameter among at least CNBD and CTBD.

In some embodiments, examples of the protein or nucleic acid, which changes (increases or decreases) expression in correlation with a morphological parameter of a nerve in an eye include, but are not limited to, cystatin-S, profilin-1, protein S100-A8, neutrophil gelatinase-associated lipocalin, phospholipid transfer protein, protein S100-A9, lipocalin-1, α1 acidic glycoprotein 1, α1-antichymotrypsin, glyceraldehyde-3-phosphate dehydrogenase, neutrophil elastase, cytokeratin 10 (Keratin, type I cytoskeletal 10), haptoglobin, protein S100-A6, paralemmin-1, laminin α5, SEC14-like protein 1, trypsin-2, secretoglobin family 1D member 1, γ actin (Actin, cytoplasmic 2), α enolase, mammaglobin-B, 14-3-3 protein ζ/δ, epithelial fatty acid binding protein, cytokeratin 75 (Keratin, type II cytoskeletal 75), histone H1.4, keratin 84 (Keratin, type II cuticular Hb4), galectin-7, cathepsin D, cytokeratin 6B (Keratin, type II cytoskeletal 6B), histone H1.1, cytokeratin 17 (Keratin, type I cytoskeletal 17), neutrophil defensin 3, cytokeratin 79 (Keratin, type II cytoskeletal 79), histone H2B type 1-N, peroxiredoxin-1, cytokeratin 12 (Keratin, type I cytoskeletal 12), transcobalamin-1, trypsin-1, lysozyme C, phosphoglycerate kinase 1, cyclophilin A (Peptidyl-prolyl cis-trans isomerase A), and any combination thereof.

In one preferred embodiment, a protein or nucleic acid, which changes (increases or decreases) expression in correlation with a morphological parameter of a nerve in an eye can be selected from the group consisting of cystatin-S, profilin-1, protein S100-A8, neutrophil gelatinase-associated lipocalin, phospholipid transfer protein, protein S100-A9, and any combination thereof.

Examples of the detection agent include, but are not limited to, antibodies that specifically bind to the protein described above, nucleic acids that specifically hybridize with the nucleic acid described above, and the like. Markers can also be detected through mass spectrometry.

In some embodiments, the detection agent of the present disclosure can be used as a diagnostic agent for a nerve abnormality in an eye. The amount of the protein or nucleic acid described above in a sample obtained from a subject is measured by using the detection agent of the present disclosure, and the measurement value is compared with a mean value of healthy individuals. If there is a significant difference in the measurement value relative to the mean value of healthy individuals, the subject can be diagnosed as having, or possibly having, a nerve abnormality in an eye. A sample can be, but is not limited to, lachrymal fluid, blood, or conjunctival scrap. In a preferred embodiment, a sample is a lachrymal fluid.

In another embodiment, the present disclosure provides a detection agent for detecting a protein or nucleic acid, which changes expression in correlation with at least one parameter selected from the group consisting of BUT, OSDI, and DEQ5.

Examples of the protein or nucleic acid, which changes (increases or decreases) expression in correlation with at least one parameter selected from the group consisting of BUT, OSDI, and DEQ5 include 14-3-3 protein θ, secretory leukocyte protease inhibitor (Antileukoproteinase), galectin-3-binding protein, α actin 1 (Actin, alpha skeletal muscle), α1-antichymotrypsin, calmodulin-3, cathepsin D, epithelial fatty acid binding protein, fibrinogen β chain, glucose-6-phosphate isomerase, histone H1.4, histone H2A type 1-C, and histone H2B type 1-J, keratin 84 (Keratin, type II cuticular Hb4), cytokeratin 79 (Keratin, type II cytoskeletal 79), laminin α5, non-histone chromosomal protein HMG-17, nucleobindin-2, peroxiredoxin-1, polymeric immunoglobulin receptor, protein S100-A7, SEC14-like protein 1, serotransferrin, thioredoxin, trypsin-2, and trypsin-3.

In a specific embodiment, the protein or nucleic acid, which changes (increases or decreases) expression in correlation with at least one parameter selected from the group consisting of BUT, OSDI, and DEQ5 can be selected from the group consisting of 14-3-3 protein θ, secretory leukocyte protease inhibitor (Antileukoproteinase), galectin-3-binding protein, and any combination thereof.

### (Diagnostic method)

In another embodiment, the present disclosure provides a method of diagnosing a condition of an eye (e.g., nerve abnormality, dry eye, or dry eye associated with a nerve abnormality) of a subject, the method comprising the steps of: obtaining a sample of the subject; measuring an amount of a marker (protein or nucleic acid) described herein in the sample; and comparing the measured amount of the marker with an amount of the marker in a sample obtained from a healthy individual to determine that the subject has a nerve abnormality in an eye based on the measured amount of the marker. The measured amount of the marker that is higher or lower than the amount of the marker in the sample obtained from the healthy individual can result in a diagnosis of having, or likely having, a condition of an eye.

In another embodiment, the present disclosure provides a method of using the marker described herein as an indicator for diagnosing a condition of an eye of a subject, comprising the steps of: measuring an amount of a marker described herein in a sample obtained from the subject; and comparing the measured amount of the marker with an amount of the marker in a sample obtained from a healthy individual.

In some embodiments, the condition of an eye can be dry eye, a nerve abnormality in an eye, a nerve abnormality in a cornea, or a nerve abnormality in dry eye. In some embodiments, a sample can be a lachrymal fluid sample.

### (Therapeutic drug screening method)

One embodiment provides a method of screening a therapeutic drug for a condition of an eye (dry eye, nerve abnormality, or the like) in a subject, the method comprising the steps of: administering a candidate agent into an eye of an animal model; and measuring amounts of a marker described herein in a sample obtained from the animal model before and after administration. If the marker in the sample obtained from the animal model after eye drop instillation is higher or lower than that before eye drop instillation, the candidate agent can be identified as effective in therapy of the condition of an eye.

The animal model used in the screening method is a non-human animal. Examples of animal models include mammals (e.g., mice, rats, hamsters, rabbits, cats, dogs, cows, horses, sheep, monkeys, and the like). In a specific embodiment, an animal model can be a lachrymal gland resected model, a model with an eye that is forced to be open, a suppressed lachrymal fluid secretion model, a corneal flap model, or a Sjogren's syndrome model.

### (Detection method)

The present disclosure provides a method of detecting a condition of an eye (e.g., nerve abnormality, dry eye, or dry eye associated with a nerve abnormality), the method comprising the steps of: measuring an amount of a marker described herein (protein or nucleic acid) in the sample; and comparing the measured amount of the marker with an amount of the marker in a sample obtained from a healthy individual. In some embodiments, a subject can be determined as having dry eye based on the measured amount of the marker. In a specific embodiment, the measured amount of the marker that is higher or lower than the amount of the marker in the sample obtained from the healthy individual can result in detection of a condition of an eye in a sample.

### (Companion diagnosis)

The present disclosure provides a method of treating a condition of an eye (e.g., nerve abnormality, dry eye, or dry eye associated with a nerve abnormality) in a subject, the method comprising the steps of: measuring an amount of a marker described herein (protein or nucleic acid) in the sample; comparing the measured amount of the marker with an amount of the marker in a sample obtained from a healthy individual to diagnose whether the subject has the condition of an eye; and administering a drug for preventing or treating a condition of an eye to a subject determined as having the condition of an eye in the step of diagnosing.

Examples of the drug for preventing or treating a condition of an eye (e.g., nerve abnormality, dry eye, or dry eye associated with a nerve abnormality) include, but are not limited to, Diquas eye drop instillation (diquafosol sodium), Mucosta eye drop instillation (rebamipide), and Hyalein eye drop instillation (purified sodium hyaluronate).

The present invention has been described while showing preferred embodiments to facilitate understanding. While the present invention is described hereinafter based on the Examples, the above descriptions and the following Examples are provided for the sole purpose of exemplification, not limitation of the present invention. Thus, the scope of the present invention is not limited to the embodiments and Examples that are specifically described herein and is limited only by the scope of claims.

### [Example]

The present invention is described more specifically hereinafter based on the Examples. The studies in the Examples are conducted in compliance with the ethical principles set forth in the Declaration of Helsinki and in accordance with the "Ethical Guidelines for Medical and Health Research Involving Human Subjects"

### (Example 1: Relationship between maintenance of homeostasis of the ocular surface and corneal sensory nerve)

The objective of this Example is to find a relationship of the morphology of a corneal sensory nerve with the corneal sensitivity and a dry eye symptom. The morphologies of corneal sensory nerves of dry eye patients and healthy adults were observed and measured using a confocal laser scanning microscope, the corneal sensitivity was measured, and ophthalmic tests were conducted on common dry eye test items, i.e., subjective symptom and ocular surface, to analyze the effect of the morphology of a corneal sensory nerve on sensitivity and its relationship with a dry eye symptom.

### (Method)

### (Target)

### Dry eye patients and healthy adults

### (Selection criteria)

This study targeted individuals who met all of the following criteria.
(1) Individuals who provided a written consent for participation in this study under their own free will
(2) Individuals who were 20 years old or older and younger than 65 years old as of the date of providing the consent (date on which the patients themselves consented to participate in the clinical trial)

### <Criteria for dry eye patients>

(3) Individuals who had a persistent subjective symptom associated with dry eye for 1 year as of the date of providing the consent
(4) Individuals with a total score of > 6 in a questionnaire related to dry eye in a screening test (DEQ-5, Japanese version)
(5) Individuals with tear film break-up time (BUT) of 5 second or less in a screening test

### <Criteria for healthy individuals>

(6) Individuals with no history of dry eye within a year prior to the date of providing the consent
(7) Individuals who were not under continuous eye drop instillation therapy for other ophthalmic diseases
(8) Individuals with a total score of ≤ 6 in a questionnaire related to dry eye in a screening test (DEQ-5, Japanese version)
(9) Individuals who fall under the following criteria in the screening test
1) Tear film break-up time (BUT) of at least ≥ 8 seconds in one eye and > 5 seconds in the other eye
2) Fluorescein staining score for keratoconjunctival epithelial disorder of < 3 points

### (Exclusion criteria)

(1) Individuals with an autoimmune disease complication including atopic dermatitis
(2) Individuals with a severe ocular disorder due to Stevens-Johnson syndrome or ocular pemphigoid
(3) Individuals with a history of ophthalmic surgery [including keratorefractive surgery (LASIK) and punctal plug insertion]
(4) Glaucoma patients (currently being treated)
(5) Individuals with a complication of anterior eye disease other than dry eye
(6) Individuals with a complication of infection
(7) Individuals who cannot discontinue or are expected to use an eye drop instillation (all prescription drugs and over-the-counter drugs except artificial lachrymal fluid) during the period from the screening test to the end of the study period
(8) Individuals who cannot discontinue the use of a contact lens from one week prior to each Visit
(9) Individuals who were determined to be unsuitable for the study by the lead investigator for the study or the like

### (Discontinuation criteria)

The lead investigator for the study or the like discontinued the study on the subjects in any of the following cases.
(1) Subject asked to withdraw from participation in the study
(2) The lead investigator for the study or the like deemed the study unsuitable

### (Study design)

### Partial blind test*, group comparison test (prospective observational study)

### *The study was conducted while concealing the pathological condition of a subject to the conductor of confocal laser scanning microscopy and image analysis

### (Flow of study)

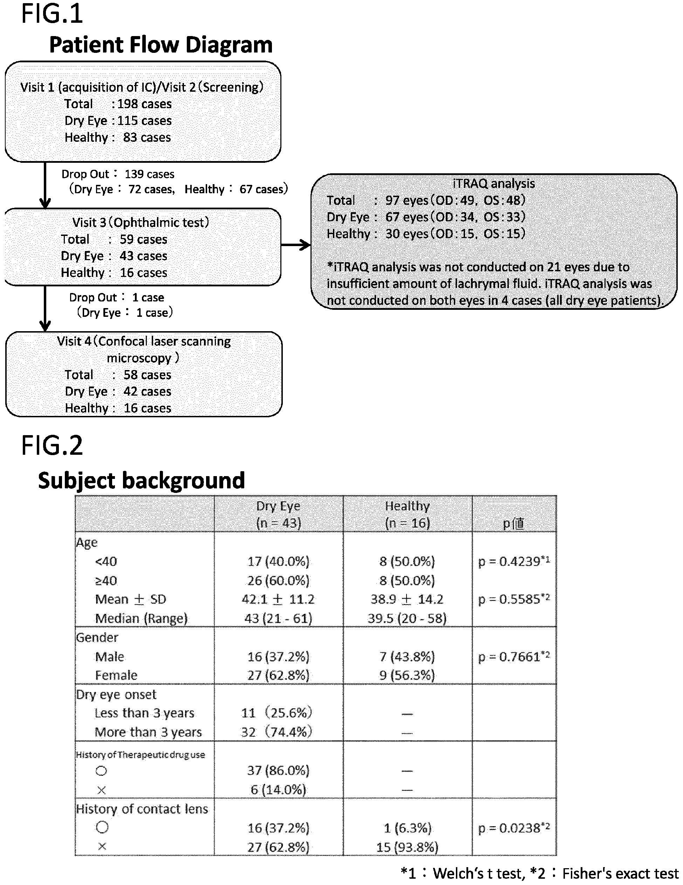

Dry eye patients and healthy adult volunteers were registered to the study to perform various observation and tests in accordance with the following procedure (Figure 1).

### (1) Visit 1

The lead investigator for the study and subinvestigator for the study of the practicing medical institution (hereinafter, the lead investigator for the study or the like) thoroughly explained the details of the study, other information related to the study, and the like to dry eye patients or healthy adults in writing and obtained a written consent for participation in the study under free will.

### (2) Visit 2

The lead investigator for the study or the like conducted a medical interview and administered a subjective symptom [used a questionnaire related to dry eye (The Dry Eye Questionnaire 5; DEQ-5 Japanese version)] and ophthalmic tests (BUT test, keratoconjunctival epithelial disorder) to all subjects from whom a written consent was obtained as a screening test to review the eligibility of the subjects. The background of the subjects is shown in Figure **2****.**

Visit 2 can also be conducted on the same day as Visit 1.

### (3) Visit 3

After Visit 2, the lead investigator for the study or the like asked the subjects to revisit the practicing medical institution and conducted lachrymal fluid collection (twice for each eye) and conducted a medical interview, subjective symptom [using questionnaire related to dry eye (DEQ-5 Japanese version, Ocular Surface Disease Index; OSDI Japanese version), questionnaire related to Neuropathic Ocular Pain (NOP), and visual analog scale (VAS) related to dry eye symptoms] and ophthalmic tests.

Two lachrymal fluid collections were conducted with an interval of 1 hour or longer, and the ophthalmic test was conducted with an interval of 1 hour or longer from the second lachrymal fluid collection. The medical interview, subjective symptom test, and investigation using a questionnaire can be conducted in any order.

### (4) Visit 4

After Visit 3, the lead investigator for the study or the like asked the subjects to revisit the practicing medical institution and conducted confocal laser scanning microscopy.

### (Indications)

### (1) Primary indication

Parameters for corneal sensory nerve based on a confocal laser scanning microscope image [Corneal Nerve Fibre Length (CNFL), Corneal Nerve Fibre Density (CNFD), Corneal Nerve Branch Density (CNBD), Corneal Nerve Fibre Total Branch Density (CTBD), Corneal Nerve Fibre Area (CNFA), Corneal Nerve Fibre Width (CNFW)]

### (2) Secondary indications

1) Parameter for neurodegeneration of the corneal sensory nerve based on a confocal laser scanning microscope image [Nerve Fibre Tortuosity (NFT)]
2) Proteins in lachrymal fluid which are comprehensively compared and quantified by iTRAQ (Example 2)
3) Tear film break-up time (BUT)
4) Schirmer's test I
5) Fluorescein staining score for keratoconjunctival epithelial disorder
6) Corneal sensitivity
7) Subjective symptom [questionnaire related to dry eye (The Dry Eye Questionnaire 5; DEQ-5 Japanese version, Ocular Surface Disease Index; OSDI Japanese version), questionnaire related to Neuropathic Ocular Pain (NOP), visual analog scale (VAS) related to dry eye symptoms]

### (Subjective symptom)

The following questionnaires were used to investigate subjective symptoms related to dry eye and neuropathic ocular pain.
(1) Questionnaire related to dry eye (DEQ-5 Japanese version) (timing of administration: Visit 2 and 3)
(2) Questionnaire related to dry eye (OSDI Japanese version) (timing of administration: Visit 3)
(3) Questionnaire related to Neuropathic Ocular Pain (NOP) (timing of administration: Visit 3)
(4) VAS related to dry eye symptoms (blurred vision, photophobia, eye irritancy) (timing of administration: Visit 3)

### (Lachrymal fluid collection)

The lead investigator for the study or the like collected lachrymal fluids for iTRAQ and for oxidative stress marker measurement twice each from both eyes for a total of four times in accordance with the following procedure during Visit 3. The second lachrymal fluid collection (oxidative stress marker) was conducted with an interval of 1 hour or longer from the first lachrymal fluid collection (iTRAQ). The results of iTRAQ are described in detail in Example 2.
(1) 50 µL of saline was administered as an eye drop to the conjunctival sac of the lower eyelid by using a sterilized micropipette.
(2) Without allowing blinking, the eye balls were moved left, right, up and down four times to mix the administered saline with lachrymal fluid.
(3) The specimen was collected in a low adsorption container from the conjunctival sac of the lower eyelid with the micropipette used for administration of saline as an eye drop.
(4) The collected specimen was quickly frozen and stored at -80°C.
(5) The cryopreserved specimen was then sent to each institution conducting measurements while being kept in a frozen state using dry ice.

### (Ophthalmic test)

The lead investigator for the study or the like conducted the following ophthalmic test.

### (1) Tear film break-up time (BUT) test (timing of administration: Visits 2 and 3)

The time from the eyelid opening until the first break of tear film in a location on the entire cornea while naturally opening the eyelid is measured three times, and the mean value thereof was calculated to the first decimal place. The breakage patterns of the tear film were also recorded under five classifications (Area break; AB, Spot break; SB, Line break; LB, Dimple break; DB, and Random break; RB).

### (2) Keratoconjunctival epithelial disorder (timing of administration: Visit 2 and 3)

In accordance with Definition and Diagnosis of Dry Eye 2006 (Jun Shimazaki, Japan Dry Eye Society, Definition and Diagnosis of Dry Eye 2006), the cornea and conjunctiva were divided into three sections (temporal bulbar conjunctiva, cornea, and nasal bulbar conjunctiva), and a staining score (fluorescein) of 0 to 3 was given to each section, and the scores were tallied.

### (3) Corneal sensitivity test (timing of administration: Visit 3)

A cornea was divided into five sections (center, top side, bottom side, temporal side, and nasal side), and the value of corneal sensitivity of each section was recorded using a Cochet-Bonnet esthesiometer. The pain tolerance at the center of the cornea was measured by using the Cochet-Bonnet esthesiometer.

### (4) Schirmer's test (timing of administration: Visit 3)

Measurements were taken by Schirmer's test I, which does not use anesthesia through eye drop instillation.

### (Confocal laser scanning microscopy)

During Visit 4, a Rostock cornea module was installed in Heidelberg retina tomograph to capture the corneal trigeminal nerve under the corneal epithelium. This was conducted while concealing the pathological condition of the subject to the conductor of confocal laser scanning microscopy.

### (Discontinuation)

The lead investigator for the study or the like discontinued the test on the subjects in any of the following cases.
(1) Subject asked to withdraw from participation in the study
(2) The lead investigator for the study or the like deemed the study unsuitable

### (Adverse event)

### (Definition of adverse event)

An adverse event was defined as any unfavorable or unintended disease, injury, or symptom thereof resulting from lachrymal fluid collection, test, or the like, regardless of the presence/absence of causal relationship with the conducted study.

### (Collection and recording of adverse event)

In case of manifestation of an adverse event, the practicing medical institution took an appropriate measure as needed, and the practicing medical institution recorded the details of the symptom and findings (name of diagnosis), presence/absence of severity, date of manifestation, date of elimination, presence/absence of treatment (details of treatment), outcome, and the like in a case record.

### (Eye targeted for evaluation)

In principle, analysis of each indication excluding subjective symptoms was directed primarily to the eye with a shorter BUT in Visit 3 for dry eye patients, and the eye with a longer BUT for healthy adults. If the BUT of both eyes was the same, evaluation was directed to the right eye.

The eye which is not the primary evaluation target of analysis was also evaluated in some cases for checking reproducibility of analysis results, exploratory analysis, or the like.

### (Criteria for handling data)

Missing values were not filled in this study. If an abnormal value was found in oxidative stress marker measurement or the like, the cause thereof was investigated, and analysis was conducted using the entire measurement data in principle, except for an abnormal value with a clear reason.

### (Analysis method)

### (Analysis of primary indication)

Summary statistics for parameters of corneal sensory nerve [Corneal Nerve Fibre Length (CNFL), Corneal Nerve Fibre Density (CNFD), Corneal Nerve Branch Density (CNBD), Corneal Nerve Fibre Total Branch Density (CTBD), Corneal Nerve Fibre Area (CNFA), and Corneal Nerve Fibre Width (CNFW)] based on a confocal laser scanning microscopic image were calculated for each dry eye patient and healthy adult, and the two groups were compared. The significance level for the hypothesis testing was 0.05 for both sides.

### (Analysis of secondary indications)

(1) A summary statistic for a parameter of neurodegeneration of a corneal sensory nerve [Nerve Fibre Tortuosity (NFT)] based on a confocal laser scanning microscopic image was calculated for each dry eye patient and healthy adult, and the two groups were compared.
(2) The difference in each protein in the lachrymal fluid quantified by iTRAQ between dry eye patients and healthy adults was studied (Example 2).
(3) A summary statistic for tear film break-up time (BUT) was calculated for each dry eye patient and healthy adult, and the two groups were compared.
(4) A summary statistic for Schirmer's test I was calculated for each dry eye patient and healthy adult, and the two groups were compared.
(5) A summary statistic for fluorescein staining score for keratoconjunctival epithelial disorder was calculated for each dry eye patient and healthy adult, and the two groups were compared.
(6) A summary statistic for corneal sensitivity was calculated for each dry eye patient and healthy adult, and the two groups were compared.
(7) Summary statistics for questionnaires related to DEQ-5 (Japanese version), OSDI (Japanese version), and NOP and results of VAS were calculated for each dry eye patient and healthy adult, and the two groups were compared.

### (Additional considerations)

In addition to analysis of primary indication and analysis of secondary indications, the relationship of the primary indication and each secondary indication with the pathological condition of a subject (dry eye patient/healthy adult) was studied by using an appropriate method such as logistic regression as an exploratory analysis. The relationship between the primary indication and each secondary indication was also studied.

### (Results)

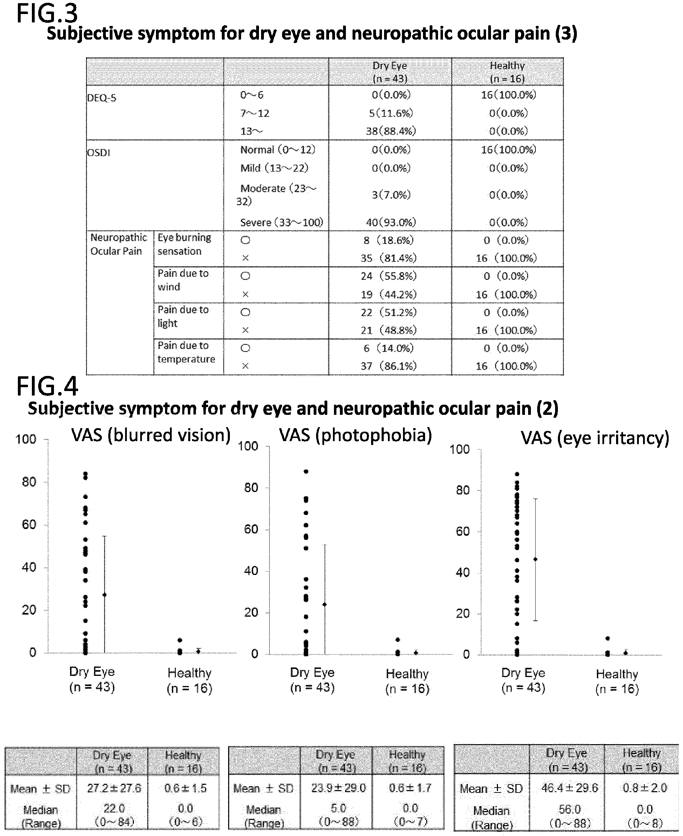

Figure **3** shows the summary of results of a questionnaire related to a subjective symptom. Healthy individuals (16 individuals) did not fall under any of the items in each question related to neuropathic ocular pain (NOP). Meanwhile, for dry eye patients, not all patients fell under each item, and some patients did not fall under any of the items. Thus, it was suggested that two types of dry eye can exist, i.e., dry eye that is associated with a nerve abnormality and dry eye that is not associated with a nerve abnormality.

Figure **4** shows a summary of results of VAS related to dry eye symptoms (blurred vision, photophobia, and eye irritancy). As shown, VAS was significantly higher in dry eye patients.

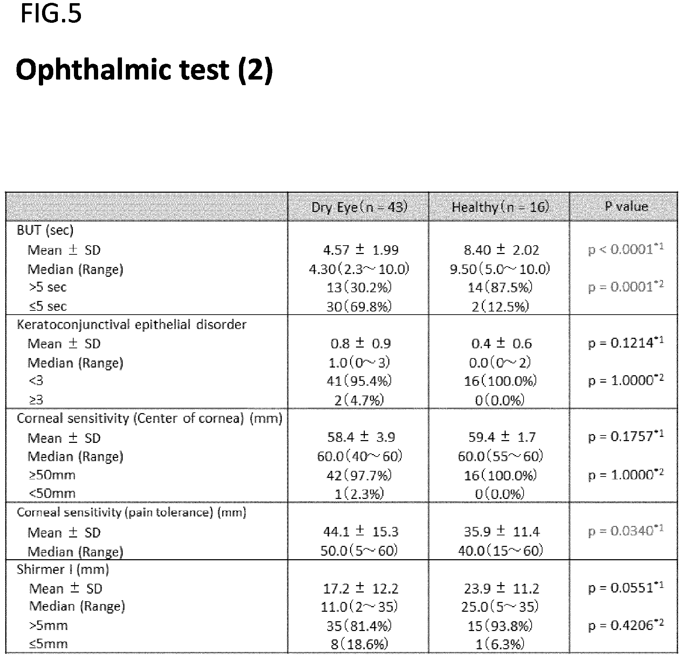

Figure **5** shows a summary of results of an ophthalmic test. BUT and pain tolerance were significantly higher in dry eye patients relative to healthy individuals, but a significant difference was not found between dry eye patients and healthy individuals in keratoconjuctival epithelial disorder, corneal sensitivity test, and Schirmer's test.

Figure **6** shows results for each nerve parameter measured by confocal laser scanning microscopy. This test measured the following parameters.
Corneal nerve fibre density (CNFD: number of major nerve fibers per 1 mm²)
Corneal nerve branch density (CNBD: number of branches on the major nerve fiber per 1 mm²)
Corneal nerve fibre length (CNFL: total length of nerve fiber per 1 mm²)
Corneal nerve fibre total branch density (CTBD: total number of branches per 1 mm²)
Corneal nerve fibre area (CNFA: total area (mm²) of nerve fibers per 1 mm²)
Corneal nerve fibre width (CNFW: mean nerve fiber width (mm) per 1 mm²)
Nerve tortuosity (mean curvature of the major fiber of the nerve in the cornea)

It was revealed that CNBD and CTBD are significantly higher in dry eye patients relative to healthy individuals. It was also demonstrated that these parameters and BUT are very highly correlated (Figure **7**).

### (Example 2: Comprehensive measurement of proteins in human lachrymal fluid)

The objective of this Example is to identify a protein correlated with a nerve parameter measured in Example 1.

### (Materials and Methods)

Lachrymal fluids obtained from 30 eyes (15 right eyes and 15 left eyes) of healthy individuals and 67 eyes (34 right eyes and 33 left eyes) of dry eye patients collected in Example 1 were subjected to measurement (Figure **1**). The lachrymal fluid was cryopreserved (-80°C) until measurement.

**[Table 1]**

| (1) Total protein concentration measurement kit | | | |
|---|---|---|---|
| Name | Micro BCA Protein Assay Kit | | |
| Manufacturer | Thermo Fisher Scientific K. K. | | |
| Cat No. | 23235 | | |
| Content | Name | Quantity | Storage condition |
| | Micro BCA Reagent A | 240 mL (2 bottles) | Room temperature |
| | Micro BCA Reagent B | 240 mL (1 bottle) | Room temperature |
| | Micro BCA Reagent C | 12 mL | Room temperature |
| | Albumin Standard: 2 mg/mL (S) | 1 mL (10 ampules) | Room temperature |

**[Table 2]**

| (2) iTRAQ reagent | | | |
|---|---|---|---|
| Name | iTRAQ Reagents-8plex One Assay Kit | | |
| Manufacturer | AB SCIEX | | |
| Cat No. | 4390811 | | |
| Content | Name | Quantity | Storage condition |
| | iTRAQ Reagents-8 plex box (113-119, 121) | 1 vial each, 1 unit/vial | -20°C |
| | Dissolution Buffer (pH 8.5) | 2 vials, 1.5 mL/vial | -20°C |
| | Denaturant | 1 vial, 50 µL/vial | -20°C |
| | Reducing Reagent | 1 vial, 100 µL/vial | -20°C |
| | Cystein-Blocking Reagent | 1 vial, 50 µL/vial | -20°C |
| | Isopropanol | 1 vial, 1.8 mL/vial | -20°C |

**[Table 3]**

| (3) Ion exchange reagent | | | |
|---|---|---|---|
| Name | CEX buffer pack | | |
| Manufacturer | AB SCIEX | | |
| Cat No. | 4326747 | | |
| Content | Name | Quantity | Storage condition |
| | Cation-Exchange Buffer-Load (Loading Buffer) | 100 mL (1 bottle) | 4°C |
| | Cation-Exchange Buffer-Elute (Eluting Buffer) | 100 mL (1 bottle) | 4°C |
| | Cation-Exchange Buffer-Clean (Cleaning Buffer) | 100 mL (1 bottle) | 4°C |
| | Cation-Exchange Buffer-Storage (Storage Buffer) | 100 mL (1 bottle) | 4°C |
| | Cation Exchange Cartridge | one | 4°C |

**[Table 4]**

| (4) Other purchased reagents | | | |
|---|---|---|---|
| Name | Grade | Manufacturer | Storage condition |
| Otsuka Normal Saline | Japanese Pharmacopoeia | Otsuka Pharmaceutical Factory, Inc. | Room temperature |
| Phosphoric acid | Special grade | Kokusan Chemical Co., Ltd. | Room temperature |
| Acetonitrile | For LC/MS | Wako Pure Chemical Industries, Ltd | Room temperature |
| Formic acid | For precise analysis | Kanto Chemical Co. Inc. | Room temperature |
| TFA | Special grade | Wako Pure Chemical Industries, Ltd | 4°C |
| Trypsin with CaC₂ (Cat No.: 4352157) | TPCK treated | AB SCIEX | -20°C |
| Remarks | Can be changed or added as needed | | |

**[Table 5]**

| Prepared reagent | | | |
|---|---|---|---|
| Name | Preparation method | Storage condition | Use-by date |
| Micro BCA solution | 25 mL Micro BCA Reagent A, 24 mL Micro BCA Reagent B, and 1 mL Micro BCA Reagent C are admixed | | Prepared upon use |
| Trypsin solution | 25 µL of water is added to trypsin with CaCl₂, and the mixture is stirred and then spun down. | | Prepared upon use |
| Water/phosphoric acid (90:10, v/v) | 9 mL of water and 1 mL of phosphoric acid are admixed | Room temperature | 2 months after preparation |
| Water/formic acid (100:0.1, v/v) | 100 mL of water and 100 µL of formic acid are admixed | Room temperature | 2 months after preparation |
| Acetonitrile/water /formic acid mixture (70:30:0.1, v/v/v) | 70 mL of acetonitrile, 30 mL of water, and 100 µL of formic acid are admixed | Room temperature | 2 months after preparation |
| Mobile phase A | 100 µL of formic acid is added to 100 mL of water | Room temperature | 2 months after preparation |
| Mobile phase B | 100 µL of formic acid is added to 100 mL of acetonitrile | Room temperature | 2 months after preparation |
| Loading solution | 100 µL of TFA is added to 100 mL of water | Room temperature | 2 months after preparation |
| Remarks | Added as needed. The amount of preparation can also be changed. | | |

**[Table 6]**

| Standard sample solution for calibration (for measuring total protein concentration) | | | | | |
|---|---|---|---|---|---|
| Name | Theoretical concentration (µg/mL) | Preparation method | | | |
| | | Standard sample solution | Amount added (µL) | Reagent | Amount added (µL) |
| W1 | 40.00 | S | 20 | Otsuka Normal Saline | 980 |
| W2 | 20.00 | W1 | 600 | Otsuka Normal Saline | 600 |
| W3 | 15.00 | W2 | 750 | Otsuka Normal Saline | 250 |
| W4 | 10.00 | W3 | 500 | Otsuka Normal Saline | 250 |
| W5 | 5.00 | W4 | 300 | Otsuka Normal Saline | 300 |
| W6 | 2.00 | W5 | 200 | Otsuka Normal Saline | 300 |
| W7 | 0 | Otsuka Normal Saline | | | |
| Remarks | Prepared upon use | | | | |
| | Polypropylene tube is used | | | | |
| | In accordance with the amount required, the prepared amount may be changed in a manner that the mixing ratio would be the same. | | | | |

**[Table 7]**

| QC sample (for measuring total protein concentration) | | | | | |
|---|---|---|---|---|---|
| Name | Theoretical concentration (mg/mL) | Preparation method | | | |
| | | Standard sample solution | Amount added (µL) | Reagent | Amount added (µL) |
| QH | 32.00 | S | 16 | Otsuka Normal Saline | 984 |
| QM | 10.00 | QH | 300 | Otsuka Normal Saline | 660 |
| QL | 3.00 | QM | 300 | Otsuka Normal Saline | 700 |
| Remarks | Prepared upon use | | | | |
| | Polypropylene tube is used | | | | |
| | In accordance with the amount required, the prepared amount may be changed in a manner that the mixing ratio would be the same. | | | | |

**[Table 8]**

| Dilution of sample (measurement of total protein concentration) | |
|---|---|
| Sample | Lachrymal fluid sample |
| Condition | Diluted with Otsuka Normal Saline |
| Preparation method | <Dilution example> 50-fold dilution: 245 µL of Otsuka Normal Saline is added to 5 µL of lachrymal fluid sample |
| Remarks | Preparation upon use |

### Measurement operation (measurement of total protein concentration)

Measurement was performed with both a standard sample solution for calibration and QC sample. The measurement sample is measured once.

**[Table 9]**

| | |
|---|---|
| 1) | 150 µL of each of a standard sample solution for calibration, QC sample, and diluted measurement sample is added to a 96-well plate (Catalog No. 3590, Corning) |
| 2) | 150 µL of Micro BCA solution is added, and the mixture is stirred for about 30 seconds. |
| 3) | The mixture is incubated for 2 hours at 37°C. |
| 4) | The 96-well plate is returned to room temperature, and absorbance at 562 nm is measured with a microplate reader. |

### Preparation of control sample

A control sample was prepared by pooling parts of each lachrymal fluid sample of healthy individuals. The lachrymal fluid samples of healthy individuals used in the pool were judged from the results of measuring the total protein concentration.

**[Table 10-1]**

| | |
|---|---|
| iTRAQ sample preparation | |
| 1) | A lachrymal fluid sample is added to a 1.5 mL tube. The amount of lachrymal fluid sample fractionated is set in the range of 5 to 100 µg protein from a result of total protein concentration, and the amount of protein added to a tube is aligned among measured samples. |
| 2) | 20 µL of dissolution buffer and 1 µL of denaturant are added. The mixture is stirred and then spun down. |
| 3) | 2 µL of reducing reagent is added. The mixture is stirred and then spun down. |
| 4) | Incubation (60°C, 1 hr) |
| 5) | 1 µL of cysteine blocking reagent is added. The mixture is stirred and then spun down. |

**[Table 10-2]**

| | |
|---|---|
| 6) | Incubation (room temperature, 10 min) |
| 7) | 10 µL of trypsin solution is added. The mixture is stirred and then spun down. |
| 8) | Incubation (37°C, overnight) |
| 9) | Spin down |
| 10) | 50 µL of isopropanol is added to each reagent of iTRAQ Reagents-8 plex (113-119, 121). The mixture is stirred and then spun down. |
| 11) | The entire amount of 10) is added to 9). The mixture is stirred and then spun down. |
| 12) | Incubation (37°C, 2 hr) |
| 13) | The entire amount of each labeled sample "113-119, 121" is transferred to a 50 mL tube and admixed. |
| 14) | Loading buffer is added at a 10-fold amount of the sample. If the pH is not 2.5 to 3.3, loading buffer or water/phosphoric acid (90:10, v/v) is further added to adjust the pH. |
| 15) | 1 mL of cleaning buffer is passed through a cation exchange cartridge. |
| 16) | 2 mL of loading buffer is passed through a cation exchange cartridge. |
| 17) | The entire amount of 14) is passed through a cation exchange cartridge. |
| 18) | 1 mL of loading buffer is passed through a cation exchange cartridge (washing). |
| 19) | 1 mL of eluting buffer is passed through a cation exchange cartridge (elution). |
| 20) | Eluate is concentrated in a centrifugal evaporator (room temperature, 90 min). |
| 21) | Water/formic acid (100:0.1, v/v) is added to 20) to about 1.5 mL. |
| 22) | Entire amount is passed through Oasis HLB µElutiοn plate (Waters) . |
| 23) | 500 µL of water/formic acid (100:0.1, v/v) is passed through. |
| 24) | 50 µL of acetonitrile/water/formic acid mixture (70:30:0.1, v/v/v) is passed through twice (elution). |
| 25) | The eluate is concentrated in a centrifugal evaporator (room temperature) until reaching about 25 µL. |
| 26) | After spin down, the eluate is dispensed in a measurement vial. |
| 27) | The vial is set in an autosampler, and measurement is repeated three times per sample. |

**[Table 11]**

| Primarily used equipment | | |
|---|---|---|
| Name | Model | Manufacturer |
| HPLC system | NanoLC-Ultra 2Dplus system | Eksigent Technologies |
| Mass spectrometer | TripleTOF 5600 | AB SCIEX |
| Microplate reader Infinite | M200 | Tecan Japan Co., Ltd |

**[Table 12]**

| List of software used | |
|---|---|
| Name | Manufacturer |
| SoftMax Pro (Ver. 5.4) | Molecular Devices Japan K.K. |
| Microsoft Excel 2010 Microsoft Excel 2016 | Microsoft Corporation |
| Analyst TF 1.6 | AB SCIEX |
| ProteinPilot 4.0 | AB SCIEX |

**[Table 13]**

| Measurement condition (iTRAQ protein measurement) High-performance liquid chromatography conditions | | | | |
|---|---|---|---|---|
| Column | ReproSil-Pur C18-AQ 3 µm 120 Å 75 µm × 15 cm (Eksigent Technologys) | | | |
| Trap column | ReproSil-Pur C18-AQ 3 µm 120 Å 200 µm × 0.5 cm (Eksigent Technologys) | | | |
| Column temperature | 35°C | | | |
| Mobile phase A | Water/formic acid (1000:1, v/v) | | | |
| Mobile phase B | Acetonitrile/formic acid (1000:1, v/v) | | | |
| Loading solution | Water/TFA (1000:1, v/v) | | | |
| Flow rate | 300 nL/min (loading solution: 2 µL/min) | | | |
| Gradient condition | (min) | A(%) | b(%) | |
| | 0.0 | 98 | 2 | |
| | 0.1 | 98 | 2 | |
| | 60.0 | 70 | 30 | |
| | 61.0 20 | | 80 | |
| | 68.0 | 20 | 80 | |
| | 68.1 | 98 | 2 | |
| | 85.0 | 98 | 2 | |
| | Linear | | gradient | |
| Sample loading | 10 minutes | | | |
| Autosampler temperature | 4°C | | | |
| Amount infused | 1 to 5 µL | | | |
| Data reading time | 85 minutes | | | |

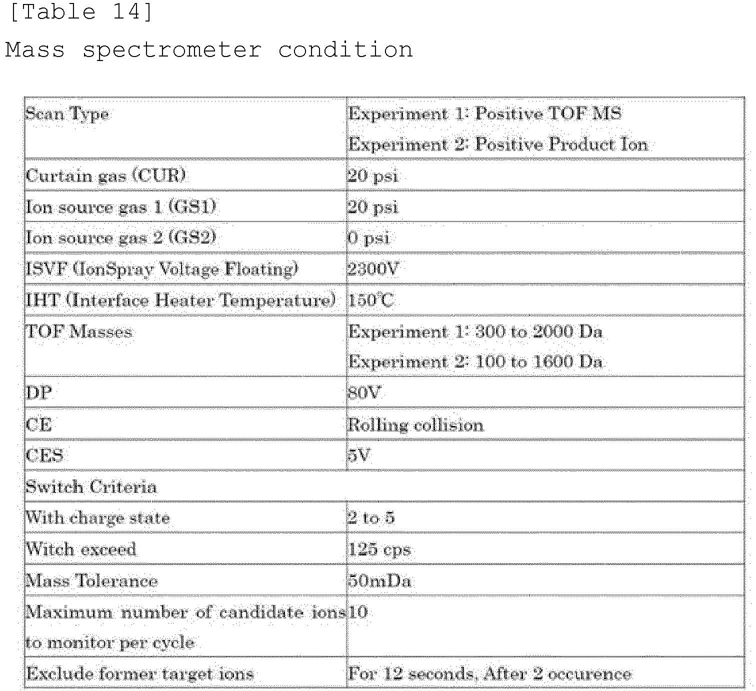

### (Results)

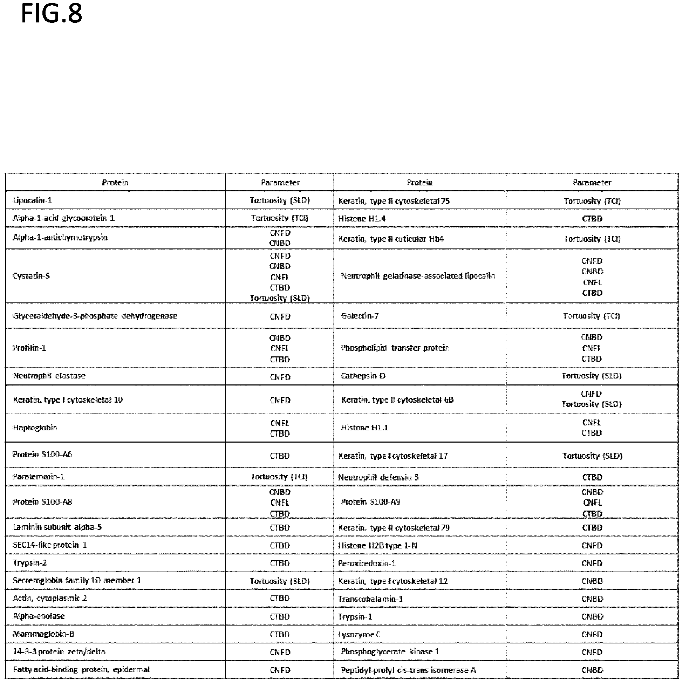

Figure **8** shows the detected proteins having a correlation with a nerve parameter (P < 0.1). Among the proteins shown in Figure **8**, cystatin-S, profilin-1, protein S100-A8, neutrophil gelatinase-associated lipocalin, phospholipid transfer protein, and protein S100-A9 in particular are correlated with both CNBD and CTBD. This demonstrates that these proteins are strongly correlated with a nerve abnormality.

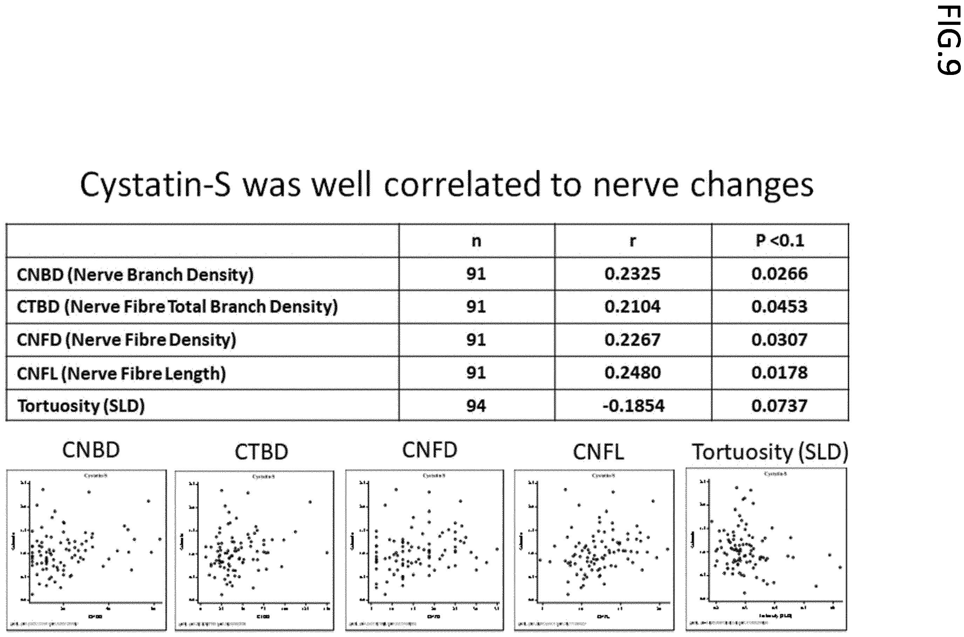

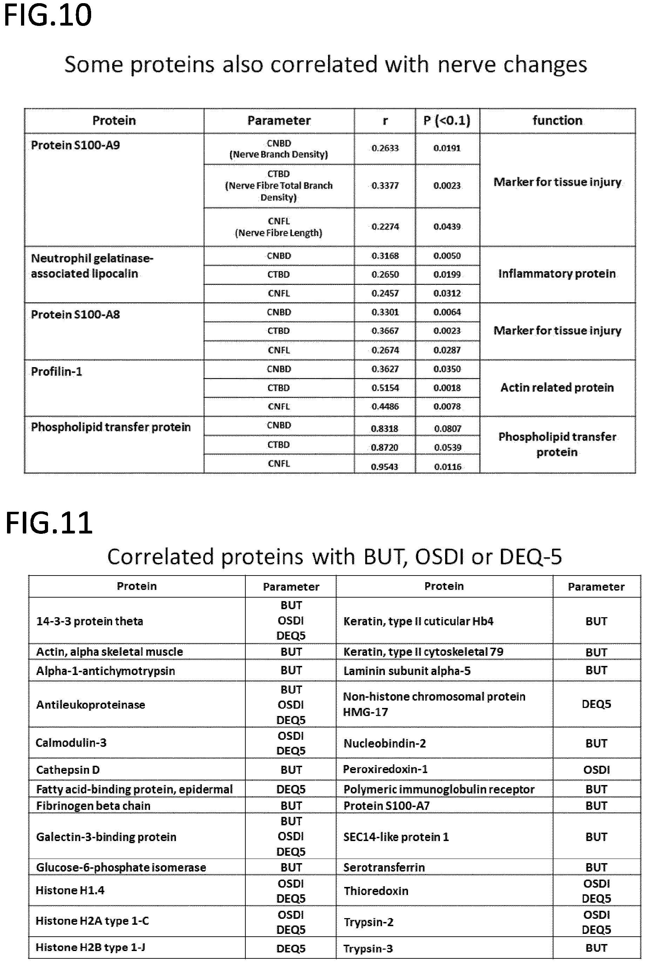

Cystatin-S is correlated with 5 parameters, i.e., CNFD, CNBD, CNFL, CTBD, and tortuosity, so that it is considered as one or the markers reflecting the condition of a nerve abnormality in an eye (Figure **9**). Similarly, profilin-1, protein S100-A8, neutrophil gelatinase-associated lipocalin, phospholipid transfer protein, and protein S100-A9 have a correlation with the corneal nerve branch density (CNBD and CTBD) and corneal nerve fibre length (CNFL), so that they are considered as markers which reflect the condition of a nerve abnormality in an eye (Figure **10**).

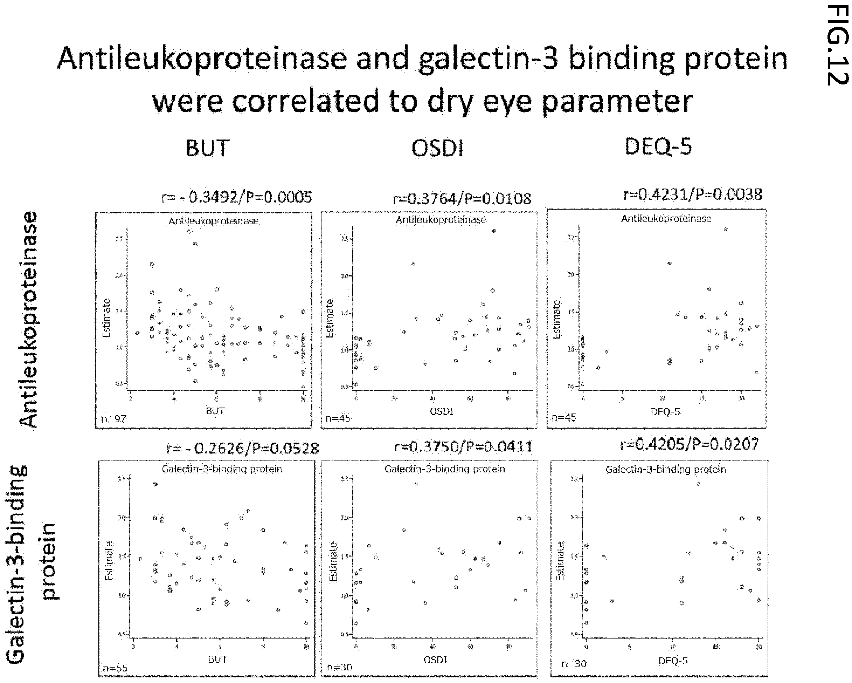

Figure **11** shows proteins correlated with BUT and subjective symptoms (DEQ5 and OSDI). In particular, 14-3-3 protein θ, secretory leukocyte protease inhibitor (Antileukoproteinase), and galectin-3-binding protein were correlated with BUT, OSDI and DEQ5. Figure **12** shows the correlation of secretory leukocyte protease inhibitor (Antileukoproteinase) and galectin-3-binding protein with each of the parameters BUT, OSDI and DEQ5. These proteins exhibited a significant correlation with each of the parameters BUT, OSDI and DEQ5.

### (Example 3: Diagnosis of nerve abnormality in eye)

Lachrymal fluid of a subject is collected, and the cystatin-S concentration in the lachrymal fluid is measured by ELISA or PCR. If the measured cystatin-S concentration in the lachrymal fluid is significantly higher or lower compared to a reference value of a healthy individual, a subject can be diagnosed as having, or likely having a nerve abnormality. In addition to cystatin-S, a marker identified in Example 2 can be further measured.

### (Example 4: Screening for an agent that treats a nerve abnormality in an eye)

A candidate agent is administered as an eye drop to a dry eye animal model such as a lachrymal gland resected model, a model with an eye that is forced to be open, a suppressed lachrymal fluid secretion model, a corneal flap model, or a Sjogren's syndrome model. A lachrymal fluid is collected before and after eye drop administration, and the concentration of cystatin-S in the lachrymal fluid before eye drop administration is compared with the concentration after eye drop administration. If the concentration of cystatin-S in the lachrymal fluid after eye drop administration is significantly changed from the concentration of cystatin-S in the lachrymal fluid before eye drop administration, the candidate agent is identified as effective in therapy of a nerve abnormality in an eye.

As described above, the present invention is exemplified by the use of its preferred embodiments. However, it is understood that the scope of the present invention should be interpreted solely based on the Claims. It is also understood that any patent, any patent application, and any references cited herein should be incorporated herein by reference in the same manner as the contents are specifically described herein.

The present application claims priority to Japanese Patent Application No. 2019-085809 filed on April 26, 2019. The entire content thereof is incorporated herein by reference.

### [Industrial Applicability]

A marker correlated with an abnormality in an eye (especially a nerve abnormality or dry eye) is provided. This marker is useful as an indicator for diagnosis or determining the efficacy of an agent and can be used in the field of pharmaceutical development or the like.

## Claims

1. A marker for a nerve abnormality in an eye, wherein the marker changes expression in correlation with at least one parameter selected from the group consisting of CNBD, CTBD, CNFD, CNFL, and tortuosity.

2. The marker of claim 1, wherein the parameter comprises at least one parameter selected from the group consisting of CNBD and CTBD.

3. The marker of claim 1 or 2, wherein the marker is selected from the group consisting of cystatin-S, profilin-1, protein S100-A8, neutrophil gelatinase-associated lipocalin, phospholipid transfer protein, protein S100-A9, lipocalin-1, α1 acidic glycoprotein 1, α1-antichymotrypsin, glyceraldehyde-3-phosphate dehydrogenase, neutrophil elastase, cytokeratin 10 (Keratin, type I cytoskeletal 10), haptoglobin, protein S100-A6, paralemmin-1, laminin α5, SEC14-like protein 1, trypsin-2, secretoglobin family 1D member 1, γ actin (Actin, cytoplasmic 2), α enolase, mammaglobin-B, 14-3-3 protein ζ/δ, epithelial fatty acid binding protein, cytokeratin 75 (Keratin, type II cytoskeletal 75), histone H1.4, keratin 84 (Keratin, type II cuticular Hb4), galectin-7, cathepsin D, cytokeratin 6B (Keratin, type II cytoskeletal 6B), histone H1.1, cytokeratin 17 (Keratin, type I cytoskeletal 17), neutrophil defensin 3, cytokeratin 79 (Keratin, type II cytoskeletal 79), histone H2B type 1-N, peroxiredoxin-1, cytokeratin 12 (Keratin, type I cytoskeletal 12), transcobalamin-1, trypsin-1, lysozyme C, phosphoglycerate kinase 1, cyclophilin A (Peptidyl-prolyl cis-trans isomerase A), and any combination thereof.

4. The marker of any one of claims 1 to 3, wherein the marker is selected from the group consisting of cystatin-S, profilin-1, protein S100-A8, neutrophil gelatinase-associated lipocalin, phospholipid transfer protein, protein S100-A9, and any combination thereof.

5. The marker of any one of claims 1 to 4, wherein the marker is a marker for a nerve abnormality in dry eye.

6. A detection agent for detecting the marker of any one of claims 1 to 5.

7. A method for identifying a marker for a nerve abnormality in an eye, comprising the steps of:
(a) obtaining a parameter selected from the group consisting of CNBD, CTBD, CNFD, CNFL, and tortuosity in a healthy individual and a dry eye patient;
(b) measuring one or more gene expression levels or protein expression levels in a sample obtained from the healthy individual and the dry eye patient; and
(c) correlating the measurement value with the one or more gene expression levels or protein expression levels.

8. The method of claim 7, wherein the parameter comprises at least CNBD or CTBD.

9. The method of claim 7 or 8, wherein the sample is a lachrymal fluid.

10. The method of any one of claims 7 to 9, wherein the marker is a marker for dry eye associated with a nerve abnormality.

11. A marker for dry eye, wherein the marker changes expression in correlation with at least one parameter selected from the group consisting of BUT, OSDI, and DEQ5.

12. The marker of claim 11, selected from the group consisting of 14-3-3 protein θ, secretory leukocyte protease inhibitor (Antileukoproteinase), galectin-3-binding protein, α actin 1 (Actin, alpha skeletal muscle), α1-antichymotrypsin, calmodulin-3, cathepsin D, epithelial fatty acid binding protein, fibrinogen β chain, glucose-6-phosphate isomerase, histone H1.4, histone H2A type 1-C, histone H2B type 1-J, keratin 84 (Keratin, type II cuticular Hb4), cytokeratin 79 (Keratin, type II cytoskeletal 79), laminin α5, non-histone chromosomal protein HMG-17, nucleobindin-2, peroxiredoxin-1, polymeric immunoglobulin receptor, protein S100-A7, SEC14-like protein 1, serotransferrin, thioredoxin, trypsin-2, trypsin-3, and any combination thereof.

13. The marker of claim 11 or 12, wherein the marker is selected from the group consisting of 14-3-3 protein θ secretory leukocyte protease inhibitor (Antileukoproteinase), galectin-3-binding protein, and any combination thereof.

14. A detection agent for detecting the marker of any one of claims 11 to 13.

15. Markers for dry eye associated with a nerve abnormality, comprising at least one marker of any one of claims 1 to 5 and at least one marker of any one of claims 11 to 13.
